(19) Europäisches Patentamt
European Patent Office
Office européen des brevets

(11) **EP 3 233 792 B1**

(12) **EUROPEAN PATENT SPECIFICATION**

(45) Date of publication and mention
of the grant of the patent:
**04.09.2019 Bulletin 2019/36**

(51) Int Cl.:
***C07C 273/04*** *(2006.01)*

(21) Application number: **15832911.0**

(86) International application number:
**PCT/NL2015/050875**

(22) Date of filing: **17.12.2015**

(87) International publication number:
**WO 2016/099269 (23.06.2016 Gazette 2016/25)**

(54) **PROCESS FOR UREA PRODUCTION**

VERFAHREN ZUR HERSTELLUNG VON HARNSTOFF

PROCÉDÉ DE PRODUCTION D'URÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2014 EP 14198947**

(43) Date of publication of application:
**25.10.2017 Bulletin 2017/43**

(73) Proprietor: **Stamicarbon B.V.
6135 KW Sittard (NL)**

(72) Inventor: **MENNEN, Johannes Henricus
6135 KW Sittard (NL)**

(74) Representative: **V.O.
P.O. Box 87930
Carnegieplein 5
2508 DH Den Haag (NL)**

(56) References cited:
**US-A1- 2011 160 486    US-B1- 6 730 811**

## Description

The present invention relates to the field of urea production. The present invention provides a urea production process, in particular a urea stripping process with a total recycle. The produced urea solutions can be used for the production of solid urea and/or solutions to be used for products such as Diesel Exhaust Fluid (DEF) which is marketed under the name AdBlue® and technical grades or used as feedstock for urea ammonium nitrate solutions and other fertilizers and resins.

## Background of the invention

Urea can be prepared by introducing an ammonia excess together with carbon dioxide at a pressure between 12 and 40 MPa and at a temperature between 150 and 250°C into a urea synthesis zone. The resulting urea formation can be presented best in the form of two consecutive reaction steps, in the first step ammonium carbamate being formed according to the exothermic reaction:

$$2NH_3 + CO_2 \rightarrow H_2N - CO - ONH_4 \qquad (1)$$

The formed ammonium carbamate is dehydrated in the second step to yield urea according to the endothermic equilibrium reaction:

$$H_2N - CO - ONH_4 \leftrightarrow H_2N - CO - NH_2 + H_2O \qquad (2)$$

The extent to which these reactions take place depends among other things on the temperature and the ammonia excess used. The reaction product obtained in a urea synthesis solution substantially consists of urea, water, unbound ammonia and ammonium carbamate. The ammonium carbamate and the ammonia are removed from the solution and are generally returned to the urea synthesis zone. In addition to the above-mentioned solution in the urea synthesis zone, a gas mixture is formed which substantially consists of unconverted ammonia and carbon dioxide together with inert gases, the so-called reactor off-gas. The urea synthesis section may comprise separate zones for the formation of ammonium carbamate and urea. These zones may also be combined in a single apparatus.

In a urea stripping plant the decomposition of the ammonium carbamate that has not been converted into urea and the expulsion of the usual ammonia excess largely takes place at a pressure that is essentially almost equal to the pressure in the synthesis reactor. This decomposition and expulsion take place in one or more stripper(s) installed downstream of the reactor, possibly with the aid of a stripping gas such as, for example, carbon dioxide or ammonia, and/or with the addition of heat. It is also possible to apply thermal stripping. Thermal stripping means that use is made exclusively of the supply of heat to decompose ammonium carbamate and remove the ammonia and carbon dioxide present from the urea solution. The gas stream leaving a thermal stripper contains ammonia and carbon dioxide which are condensed in a high pressure condenser and then returned to the synthesis section.

In a urea stripping plant the synthesis section is typically operated at a temperature of 160 - 240 °C and preferably at a temperature of 170 - 220 °C. The pressure in the urea synthesis reactor is preferably 12 - 21 MPa, more preferably 12.5 - 20 MPa. The ammonia to carbon dioxide molar ratio (N/C ratio) in the synthesis section of a stripping plant lies usually in the range 2.2-5 and preferably in the range 2.5-4.5 mol/mol. The urea synthesis can be carried out in a single reactor or in a plurality of reactors.

After the stripping treatment, the pressure of the stripped urea solution is reduced in the urea recovery section and the urea solution is concentrated by the evaporation of water. The produced carbamate stream formed in the recovery section operated at a lower pressure than the pressure in the synthesis section, is preferably returned to the urea synthesis section operating at synthesis pressure. The recovery section can be carried out in a single section or in a plurality of recovery sections arranged in series.

The energy consumption of urea stripping plants is generally determined by the steam consumption. Steam is usually supplied to the stripper shell in the synthesis section solely or to both the stripper shell and the heater shell in the downstream urea recovery section.

A known urea stripping production process is the Snamprogetti ammonia stripping process as for example described in GB Patent 1,188,051 published on April 15, 1970 and described in Ullmann's Encyclopedia of Industrial Chemistry, Vol. A27, 1996, pp. 333-350. The carbon dioxide feed in such a process is usually completely added to the reactor. The carbon dioxide feed contains inert vapor and often air is supplied to carbon dioxide to keep the materials used in the synthesis resistant against excessive corrosion. The urea solution leaving the reactor containing urea, water and non-converted ammonium carbamate together with the inert vapor that contains non-converted ammonia and carbon dioxide gas are supplied after flashing to the high-pressure stripper. The high-pressure stripper is a shell and tube type of heat exchanger. At the shell side, steam is supplied that provides the required heat necessary for the decomposition reaction of ammonium carbamate into ammonia and carbon dioxide vapor. At the top of the tube side, the urea solution is added and the liquid flows as a liquid film along the tube to the bottom channel of the stripper. The released vapor by the decomposition of the ammonium carbamate reaction leaves the heat exchanger tube at the top and is sent to the carbamate condenser that is usually a kettle type. In this process the stripper is typically operated at a pressure about 10 bar lower than the pressure in the urea reactor in order to improve the stripper efficiency. This leads to the need for an ejector to increase the pressure of the carbamate solution prior to recycle to the reactor.

US2011/0160486A1 describes a urea production system with an isobaric loop, a submerged condenser and using carbon dioxide as stripping gas.

US 6730811B1 discloses a similar production system but the loop is not isobaric

Another example of a known process is described in CA975797. In this process the reaction takes place in two reactors and the stripping is carried out in a single step with a gas which is inert to the initial reactants or to ammonium carbamate (*i.e.* not ammonia or carbon dioxide). The gas is recycled to the reactor and either vented or again used for stripping. As inert gases ammonia synthesis gases namely nitrogen and hydrogen are used. Disadvantages of this process are the use of the very flammable hydrogen and the fact that the inert gases take up reactor space thereby increasing the reactor size for a given urea production.

It is desired to provide a urea production process with reduced investment costs and reduced energy consumption. It is also desired to provide a process with a high conversion of $CO_2$ and $NH_3$ in the synthesis cycle. It is further desired to design a process that efficiently uses the reactor volume.

## Brief description of the drawings

Figure 1 shows a process scheme for a urea stripping process according to prior art using carbon dioxide as a stripping agent.
Figure 2 shows a process scheme for a urea stripping process according to prior art wherein heat stripping is used.
Figure 3 shows a process scheme for an embodiment according to the present invention, wherein the reactor and the condenser are combined in one apparatus.
Figure 4 shows a column in an aerosol stripper.
Figure 5 shows a process scheme for an embodiment according to the present invention, wherein the reactor and the condenser are separate pieces of equipment.

## Brief description of the invention

In order to address at least some of the above-mentioned needs, the present invention, in one aspect, provides a process for urea production in a urea plant comprising a synthesis section, said section comprising a urea reactor, a stripper, a condenser and optionally a scrubber, and a recovery section operating at lower pressure than the synthesis section, said process comprising the steps of:

a) reacting ammonia and $CO_2$ under urea forming conditions in the urea reactor to obtain a first urea solution,
b) stripping the first urea solution with ammonia in the stripper to form a second urea solution and a first vapor phase containing ammonia, $CO_2$ and water,
c) condensing the first vapor phase obtained in b) in the condenser to form a carbamate solution, which is recycled to the reactor,
d) subjecting the second urea solution to ammonia recovery in the recovery section to produce a third urea solution and a second vapor phase containing ammonia,
e) recycling the second vapor phase to the urea synthesis section,

wherein in the synthesis section a substantially isobaric loop is formed,
wherein the second urea solution contains less than 3 wt.%, preferably less than 2 wt.%, more preferably less than 1 wt.% of $CO_2$, and
wherein the condenser is a submerged condenser.

## Detailed description of the invention

The invention is based on the judicious insight that it is possible to increase the overall conversion and reduce operating costs by carrying out a urea process with substantially complete $CO_2$ conversion thereby eliminating the need for carbamate recycle from the recovery section(s). Carbamate recycle from the recovery section(s) is common in all existing urea processes and brings about a disadvantage of a significant recycle of water to the reactor. Water recycled to the reactor has a negative influence on the conversion and efficiency of urea production.

In the present invention a substantially complete $CO_2$ conversion is achieved in the synthesis section and substantially only ammonia is recycled to the reactor thereby improving the overall conversion and urea production productivity. This is achieved by increasing the $CO_2$ conversion in the reaction zone of the urea synthesis. The increase in the $CO_2$ conversion is obtained because the N/C molar ratio in the reaction zone is substantially higher than compared to the applied N/C molar ratios in the synthesis sections of usual urea stripping processes and because the pressure in the synthesis section is substantially higher than compared to the pressures as applied in the usual stripping processes. The N/C molar ratio in the urea synthesis section is preferably in the range 3.8 - 4.2 mol/mol and more preferably in the range 3.9 - 4.1 mol/mol. The pressure in the synthesis section is preferably in the range 18 to 21 MPa and more preferably in the range 18.5 - 20.5 MPa. The process conditions in combination with a proper achieved stripping efficiency in which ammonia is used as a stripping agent makes that the residual carbon dioxide in the urea solution leaving said stripper is negligible. Therefore there is no need for the usually applied carbamate recycle streams which are a necessary element for the competing stripping processes.

The present invention provides a stripping process to produce urea. Stripping processes are known to a skilled person.

A frequently used process for the preparation of urea according to a stripping process is the carbon dioxide stripping process as for example described in Ullmann's Encyclopedia of Industrial Chemistry, Vol. A27, 1996, pp. 333-350. In this process, the synthesis section is followed by one single recovery section or more recovery sections. The synthesis section comprises a stripper, a condenser, a reactor and a scrubber in which the operating pressure is in between 12 and 18 MPa and preferably in between 13 and 16 MPa. In the synthesis section the urea solution leaving the urea reaction zone is sent to a high pressure stripper in which a large amount of non-converted ammonia and carbon dioxide is separated from the aqueous urea solution. Such a stripper is typically a shell and tube heat exchanger, in which the urea solution is fed to the top part at the tube side and the carbon dioxide feed to the synthesis is added to the bottom part at the tube side of the stripper. At the shell side, steam is added to heat the solution. The urea solution leaves the heat exchanger at the bottom part while the vapor phase leaves the stripper at the top part.

The vapor leaving said stripper contains ammonia, carbon dioxide and a small amount of water. Said vapor is condensed in a high pressure carbamate condenser, which is typically a falling film type heat exchanger or a submerged type of condenser that can be of a horizontal type or a vertical type. A horizontal type submerged heat exchanger is described in Ullmann's Encyclopedia of Industrial Chemistry, Vol. A27, 1996, pp 333-350. The released heat by the exothermic carbamate condensation reaction in said condenser is usually used to produce steam that is used in the downstream processing of the urea plant for heating and concentrating the urea solution. Since a certain liquid residence time is created in a submerged type condenser, a part of the urea reaction takes already place in said condenser.

The solution formed in the condenser contains condensed ammonia, carbon dioxide, water and urea and, together with the non condensed ammonia, carbon dioxide and inert vapor, it is sent to the reactor. In the reactor the above mentioned reaction from carbamate to urea approaches the equilibrium. The ammonia to carbon dioxide molar ratio in the urea solution leaving the reactor is generally in the range 2.5 and 4 mol/mol but in practice in between 2.8 and 3.2 mol/mol. It is also possible that the condenser and the reactor are combined in one piece of equipment. An example of this piece of equipment as described in Ullmann's Encyclopedia of Industrial Chemistry, Vol. A27, 1996, pp 333-350.

If a scrubber is present, the formed urea solution leaving the urea reactor is supplied to the stripper and the inert vapor containing non-condensed ammonia and carbon dioxide is sent to a scrubber operating at a similar pressure as the reactor. In the scrubber the ammonia and carbon dioxide are scrubbed from the inert vapor. The formed carbamate solution from the downstream recovery system is used as absorbent in the scrubber.

The urea solution leaving the stripper in the synthesis section requires a urea concentration of at least 50 wt.% and preferably at least 53 wt.% to be treated in one single recovery system downstream the stripper. The amount of non-converted $CO_2$ in this solution is typically 10 to 12 % by weight. The recovery section typically comprises a heater, a liquid/gas separator and a condenser. The pressure in this recovery section is typically in the range 0.2 to 0.5 MPa. In the heater of the recovery section the bulk of ammonia and carbon dioxide is separated from the urea and water phase by heating the urea solution. Usually steam is used as a heating agent. The urea and water phase that contains a small amount of dissolved ammonia and carbon dioxide, which leave the recovery section, are sent to a downstream section where the urea solution is concentrated by evaporating the water from said solution. The vapor released in the heater comprises ammonia, carbon dioxide and water. Said vapor is condensed in a condenser. The heat of condensation is dissipated in cooling water. The formed carbamate is recycled to the synthesis section and can be used as absorbent in said scrubber in the synthesis section. Some non-condensed vapor leaving that condenser is sent to a condenser or absorber in order to purify the inert before releasing it into the atmosphere.

The process according to the invention takes place in a urea plant comprising a synthesis section and at least one recovery section. The synthesis section comprises a urea reactor, a stripper, a carbamate condenser and optionally a scrubber. The synthesis section operates at high pressure, while the recovery sections operate at a lower pressure than the synthesis section. In the present invention, the urea reactor, carbamate condenser and the stripper in this synthesis section operate at a nearly equal pressure. In other words, it can be said that a substantially isobaric loop is formed in the synthesis section to produce a urea reaction solution.

In the process according to the invention, ammonia and $CO_2$ are reacted under urea forming conditions in the urea reactor to obtain a first urea solution. This urea solution is also called urea reaction solution. In one embodiment, the urea reactor and the urea condenser are preferably combined in one apparatus. The N/C ratio during the reaction is preferably in the range 3.8-4.2, more preferably in the range 3.9-4.1. The pressure in the reactor is preferably in the range 18-21 MPa, more preferably 18.5-20.5 MPa. The temperature of the urea solution leaving the reactor is preferably in the range 180-200 °C. These conditions contribute to the low $CO_2$ content in the following step.

In step (b), this solution is stripped with ammonia in the stripper to form a second urea solution and a first vapor phase containing ammonia, $CO_2$ and water. The second urea solution is a stripped urea reaction solution and contains less than 3 wt.%, preferably less than 2 wt.%, more preferably less than 1 wt.% of $CO_2$. The particular $CO_2$ concentration in the second urea solution is a clear directive to the skilled person who knows how this can be achieved. The $CO_2$ content under 3 wt.% has an effect that the carbamate recycle from a recovery section to the synthesis section, being typical for urea stripping total recycle plants, is absent. Consequently, the absence of the carbamate recycle encourages the urea reaction conversion in the synthesis section resulting in a reduced operational cost for the produced urea product.

The stripping in step (b) can be done, as usual in total recycle urea stripping processes, in a falling film type heat exchanger, wherein the urea solution arrives at the top and is distributed over a number of tubes. The urea solution contacts the stripper tube wall as a liquid film. Around the tubes steam is added and supplies the required heat to the urea solution film by conduction. By this heating, ammonia and carbon dioxide present in the urea solution evaporate and leave the shell and tube stripper via the top while the urea solution leaves the stripper via the bottom. Ammonia can be supplied to the stripper bottom and is used as a stripping agent to ease the decomposition of the carbamate in the urea solution.

A preferred stripper to be used in the process according to the present invention is a so-called aerosol stripper. In this stripper the added urea solution is dispersed in the continuous gas phase. The required vapor in order to obtain the aerosol in the stripper is produced by adding heat to the urea solution to be stripped. The heat supplied reaches the liquid by conduction as well as by convection and decomposes the remaining carbamate in the urea solution into carbon dioxide and ammonia, which form the continuous gas phase. The aerosol stripper improves the heat and mass transfer of the decomposition of non-converted ammonia and carbon dioxide in the urea solution and consequently the residual carbon dioxide in the urea solution leaving such an aerosol stripper is about 1 % by weight lower as compared to the residual carbon dioxide content as observed in the urea solution effluent leaving the usual applied falling film type strippers. When an aerosol stripper is used, the amount of carbon dioxide in the urea solution effluent leaving the stripper is almost negligible ($\leq$ 3 % by weight) and thus the carbamate recycle from one or more downstream recirculation sections that is usually applied in total recycle urea plants is omitted. Since at least a part of the ammonia is added to the bottom of the stripper, the urea solution effluent leaving said stripper contains almost zero biuret. The biuret content in this solution is at least 0.1 %, preferably at least 0.05 % by weight lower than the biuret content in urea solutions leaving the stripper in $CO_2$ stripping plants.

The use of ammonia stripping, particularly in an aerosol stripper, ensures almost zero biuret content in the solution leaving the stripper which is an advantage as it improves the quality of the final product. This is particular advantageous when the plant is run at lower than 100% capacity, because in prior art processes, the level of biuret in the solution leaving the stripper will typically be increased when the stripper is not run at full capacity which may lead to problems with the final product quality.

Other advantages of ammonia stripping as used in the present process over a $CO_2$ stripping process include the low residual $CO_2$ in the stripper effluent resulting in a negligible carbamate recycle as compared to the known $CO_2$ stripping and ammonia stripping processes. Consequently the recycled water (by the carbamate recycle stream) to the synthesis section is negligible as well and since the urea reaction is a hydration equilibrium reaction the achieved urea conversion in this process is much higher as compared to the known $CO_2$ stripping and ammonia stripping processes that reduces the involved OPEX considerably.

Carbamate solution comprises next to the components ammonia and carbon dioxide, the component water necessary to keep the carbamate flow in the liquid phase that is needed to convey such a carbamate flow to a higher pressure by means of *e.g.* a pump. The recycled carbamate flow is formed in the downstream recovery section of the urea synthesis section and is returned as a liquid to the synthesis section operating at elevated pressure. The amount of water, necessary to keep the carbamate in the liquid phase, depends generally on the ammonia to carbon dioxide molar ratio in the carbamate, the temperature and the pressure where condensation of ammonia and carbon dioxide into carbamate solution takes place. In common urea stripping processes the recycled carbamate flow is fed to the synthesis section where the endothermic urea equilibrium reaction occurs. The amount of water entering that synthesis section by such a carbamate recycle flow is the dominant parameter for the determination of the chemical urea equilibrium of the endothermic urea reaction in said synthesis section.

This principle that the amount of water affects the chemical equilibrium can be illustrated by plotting the conversion against the water to $CO_2$ ratio. Such diagrams are for example published in the Ullmann's Encyclopedia of Industrial Chemistry, Vol. A27, 1996, pp. 336-338, wherein the $CO_2$ conversion or the $NH_3$ conversion, respectively, is plotted against the $H_2O/CO_2$ ratio. The chemical equilibrium is expressed in respectively the $CO_2$ conversion and the $NH_3$ conversion. The $CO_2$ conversion relates to the relative conversion rate of urea in which the present carbon dioxide is used as a reference. In the ammonia conversion the present ammonia is used as a reference. The $CO_2$ conversion in the urea solution leaving the reactor is defined as:

$$CO_2 \text{ conversion} = 100\% * (Urea/M_{Urea})/(Urea/M_{Urea} + CO_2/M_{CO2})$$

The ammonia conversion in the urea solution leaving the reactor is defined as:

$$NH_3 \text{ conversion} = 100\% * (2*Urea/M_{Urea})/(2*Urea/M_{Urea} + NH_3/M_{NH3})$$

The water to carbon dioxide molar ratio is the molar ratio between the amount of water and of carbon dioxide in the urea solution leaving the reaction zone in the urea synthesis section. Said water to carbon dioxide molar ratio is defined as follows:

$$H_2O/CO_2 = (Water/M_{H2O} - Urea/M_{Urea})/(Urea/M_{Urea} + CO_2/M_{CO2}),$$

wherein $M_{H2O}$ is the molar weight of water, $M_{Urea}$ is the molar weight of urea, $M_{CO2}$ is the molar weight of carbon dioxide and $M_{NH3}$ is the molar weight of ammonia.

When the $CO_2$ or $NH_3$ conversion is plotted against the $H_2O/CO_2$ ratio, a linear relationship is obtained. In both cases, the diagrams show that increasing the $H_2O/CO_2$ ratio (and hence the amount of water to the synthesis section) decreases the amount of urea formed at chemical equilibrium of the endothermic urea reaction in the synthesis section. These relationships teach that the absence of a carbamate flow from the recovery section to the urea synthesis process results in a decrease of water arriving to the synthesis section via these recycle streams and thus favors the chemical equilibrium of the endothermic urea reaction. Preferably, the water to $CO_2$ ratio in the second urea solution is in the range 0.2-0.45 mol/mol and more preferably in the range 0.25-0.4 mol/mol.

In one preferred embodiment, the second urea solution is subjected to flashing under medium pressure to obtain a flashed urea solution and an ammonia containing vapor. Preferably, the second urea solution is cooled by indirect heat exchange with said flashed urea solution.

In step (c), the first vapor phase is condensed in the condenser to form a carbamate solution which is recycled to the reactor. This condenser is a submerged condenser. Consequently by the retention, substantial urea formation takes already place in this condenser zone of the synthesis section. Preferably, the submerged condenser is of a horizontal type, which is also typically called pool condenser. The carbamate solution preferably comprises 10 to 30 % by weight of converted urea.

The second urea solution is subjected in step (d) to ammonia recovery in the recovery section to produce a third urea solution and a second vapor phase containing ammonia. In step (e) the second vapor phase, which contains at least 90 % by weight of ammonia, is recycled, preferably together with the ammonia feedstock, to the urea synthesis section.

Preferably, the driving force in the urea synthesis loop is gravity flow and, in one preferred embodiment, all the flows in the synthesis section are gravity flows. Preferably, at least a part of the ammonia flow is added to the bottom part of the high-pressure stripper in this synthesis loop.

The invention will further be illustrated on the basis of the following, non-limiting examples.

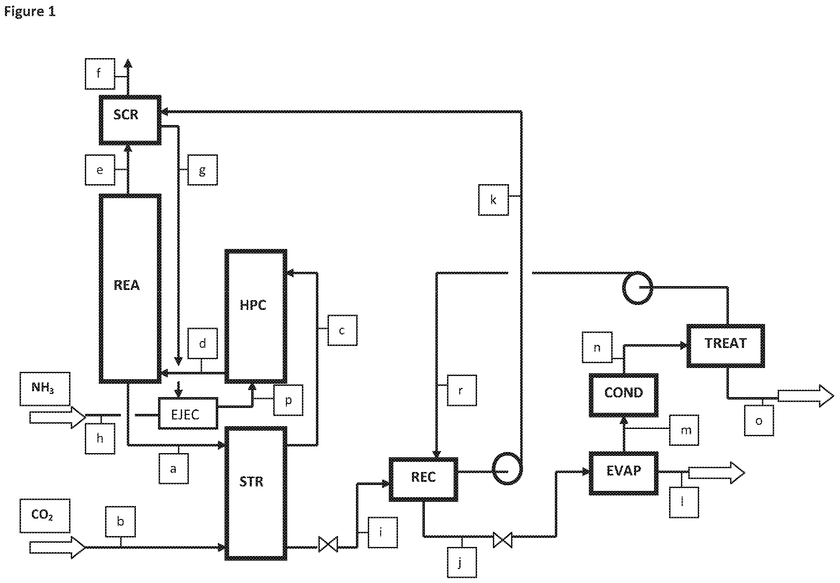

Figure 1 represents a known urea stripping process using carbon dioxide as a stripping agent.

The high pressure synthesis section comprises a reactor (REA), a stripper (STR), a condenser (HPC) and a scrubber (SCR) that comprises a scrubber bed and/or a heat exchanger. The synthesis section is typically operated at a pressure in between 13 and 16 MPa and the equipment in the synthesis are in fluid communication. The synthesis process loop involves gravity flows.

In the synthesis section the urea solution leaving the urea reactor (REA) is fed (a) to a stripper (STR) in which a large amount of non-converted ammonia and carbon dioxide is separated from the aqueous urea solution. The stripper is a shell and tube heat exchanger in which the urea solution is fed to the top part via line (a) at the tube side and a carbon dioxide feed is added to the bottom part of the stripper via line (b) at the tube side. The carbon dioxide may but not necessarily contain inert and may comprise air for protecting the fabrication materials of the equipment and lines in the synthesis section against excessive corrosion. The urea solution from the reactor (REA) supplied to the stripper (STR) via line (a) is counter currently contacted with the supplied carbon dioxide. By this the partial pressure of ammonia in the urea solution is decreased, which causes the non converted carbamate to decompose. As a heating agent, steam at a pressure of 1.5 to 2.5 MPa is supplied to the shell side of said stripper (STR) in order to obtain a urea concentration in the urea solution leaving that stripper of approximately 53 to 56 % by weight. The vapor leaving the stripper (STR) via line (c) contains ammonia, carbon dioxide, inerts and a small amount of water and is supplied to a condenser (HPC). Ammonia is supplied to that high pressure condenser (HPC) as well, via line (p). In this condenser the ammonia and carbon dioxide is condensed into a carbamate solution. The released condensation heat is used to form steam that is used for heating purposes in the downstream urea processing sections. If the condenser (HPC) is of a submerged type, residence time of the liquid phase is created, which causes the endothermic urea reaction to proceed. The released condensation heat is used to form steam that is used in the downstream processing of the synthesis agent as heating agent and driving force for ejectors. The thus formed solution together with non-condensed inert vapor leaving the condenser (HPC) is sent to the reactor (REA) via line (d) where the endothermic urea reaction approaches the equilibrium. In the top of the reactor (REA) the solution is separated from the non-condensed vapor that comprises next to ammonia and carbon dioxide also inerts. The urea solution leaving the reactor (REA) is typically controlled at an ammonia to carbon dioxide molar ratio in the range of 2.8 to 3.2 mol/mol and the water to carbon dioxide molar ratio in the urea solution leaving the reactor (REA) is typically in the range of 0.45 to 0.7 mol/mol and usually in the range 0.5 to 0.65 mol/mol. The non-condensed inert vapor leaving the reactor (REA) is sent to the scrubber (SCR) via line (e). In the scrubber (SCR) the non-condensed ammonia and carbon dioxide is separated from the inert vapor by feeding the carbamate, formed in the recovery section as absorbent via line (k). The inert vapor via line (f) is sent into the atmosphere directly or is treated in an absorber before releasing it into the atmosphere. The formed carbamate solution in the scrubber (SCR) is returned to the condenser (HPC) via line (g). In most cases the formed carbamate solution in the scrubber (SCR) is elevated in pressure by an ammonia ejector (EJEC) before it enters the condenser (HPC) via line (p). As a driving force for this ejector (EJEC) the liquid ammonia feedstock is used that enters said ejector via line (h).

The urea solution leaving the urea stripper (STR) via line (i) comprises typically in between 9 and 14 % by weight of non-converted carbon dioxide and is expanded and sent directly to a recovery section (REC). The operating pressure in the recovery section is usually 0.2 to 0.6 MPa. The recovery section comprises usually a decomposer and a condensation. The decomposer is usually a shell and tube heat exchanger. In the decomposer the carbamate left in the urea solution is decomposed into ammonia and carbon dioxide vapor by adding steam to the shell side. The urea solution leaves said decomposer at a temperature of 120 - 145 °C and preferably at a temperature of 130 to 140 °C after which the pressure of said urea solution is decreased causing a further purification of the urea solution by flashing.

The released vapor from the decomposer comprising ammonia, carbon dioxide and water is condensed in the condensation section of this recovery section (REC) by the formation of a carbamate solution that is pumped to preferably but not necessarily the scrubber (SCR) in the synthesis section.

The urea solution leaving the recovery section (REC) arrives via line (j) in the evaporation section (EVAP). In this evaporation section (EVAP) the urea solution is concentrated to the desired urea concentration that is determined by the finishing section (not shown). Usually the concentration of the urea solution takes place at sub-atmospheric pressure and dependent on the required concentration one or a plurality of evaporators are used. The concentrated urea solution leaves the evaporation section via line (l). The released vapor leaving the evaporators via line (m) comprising water, ammonia and carbon dioxide is condensed to form process condensate. Since the operating pressure in the evaporator is sub-atmospheric, small amounts of urea are entrained and leave the evaporators via the vapor phase. This urea is traced back in the formed process condensate leaving the condensation section (COND) via line (n). Said formed process condensate is after totally or partially being used as absorbent in the absorbers of the plant to purify inert vapor, subjected to a process condensate treatment section (TREAT). The process condensate treatment section comprises a first desorber, a hydrolyser column followed by a next second desorber (not shown). In the first desorber the bulk of ammonia and carbon dioxide is stripped from the water solution. The stripped condensate is subjected to a heated liquid filled column (hydrolyser) in which the urea, occurring in said condensate, is decomposed into ammonia and carbon dioxide. The effluent leaving this hydrolyser comprising water, ammonia and carbon dioxide is subjected to the next second desorber in which the residual ammonia and carbon dioxide is stripped from the condensate. Usually steam is used as stripping agent in this second desorber. The purified process condensate leaves the treatment section (TREAT) via line (o). The hot off gas leaving the second desorber comprising ammonia and carbon dioxide is usually used as stripping agent for the said first desorber. The off gases leaving the first desorber, comprising ammonia, carbon dioxide and water, are condensed where after that formed concentrated process condensate is added via line (r) to the condensation section of the recovery section (REC).

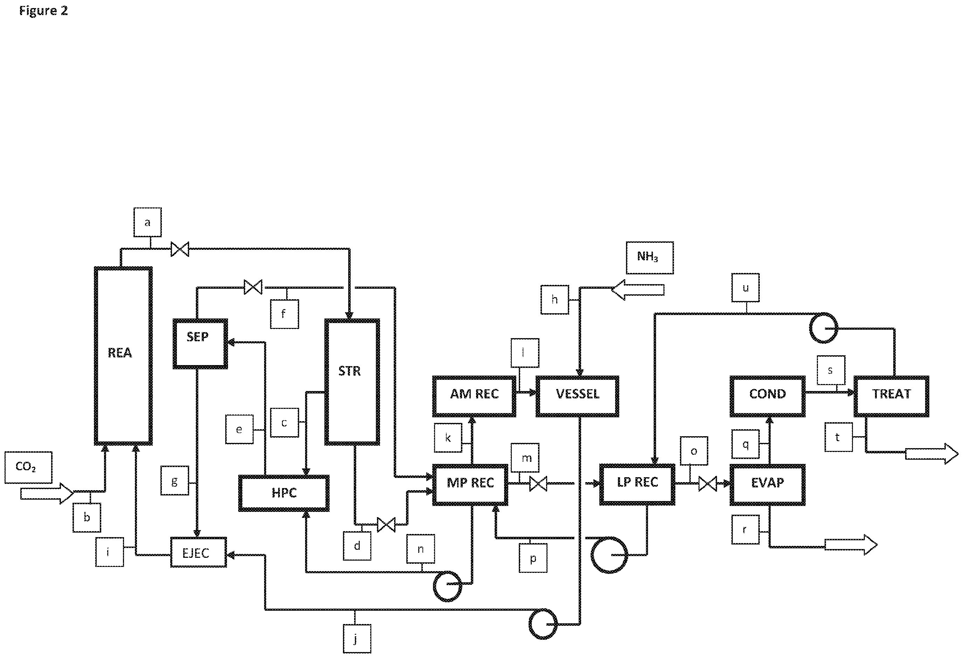

Figure 2 shows a typical heat stripping process known in the art. The synthesis section comprises typically the equipment: a reactor (REA), a stripper (STR), a condenser (HPC), an ejector (EJEC) and a liquid/gas separator (SEP). The reaction zone (REA) in the synthesis section is typically operated at a pressure in between 15 and 19 MPa while the pressure in the reactor (REA) is typically 0.3 to 1.2 MPa higher than the pressure in the remaining synthesis equipment. The pressure difference is realized by the application of an ejector (EJEC) that boosts the carbamate solution, formed in the condenser (HPC). The condenser (HPC) is typically a kettle type and since the tube side is the process side in this condenser, is the retention limited and as a consequence is the formed urea in such a condenser negligible. As driving force pressurized ammonia is used in the ejector (EJEC).

In the synthesis section the urea solution leaving the urea reactor (REA) is typically controlled at an ammonia to carbon dioxide molar ratio in between 3.1 and 3.9 mol/mol and the water to carbon dioxide molar ratio in the urea solution leaving the reactor (REA) is typically in the range of 0.5 to 0.7 mol/mol and usually in the range of 0.55 to 0.65 mol/mol. This urea solution is fed to a stripper (STR) in which a large amount of non-converted ammonia and carbon dioxide is separated from the aqueous urea solution. The stripper is a shell and tube heat exchanger in which the urea solution is fed to the top part via line (a) at the tube side. At the shell side of said stripper steam at a pressure of 1.0 to 2.3 MPa is added as a heating agent. The released vapor leaving the stripper (STR) via line (c) is sent to the condenser (HPC) together with the carbamate solution formed in the downstream recovery section (MP REC) via line (n). In this condenser (HPC) the exothermic carbamate reaction takes place. The released condensation heat is used to form steam that is used as heating agent and driving force of ejectors in the downstream processing of the synthesis section. The formed carbamate solution together with non-condensed ammonia and carbon dioxide vapor is sent via line (e) to a vessel (SEP) in which the vapor phase is separated from the carbamate liquid phase. The carbamate solution is sent via line (g) to an ejector where the discharged carbamate solution is increased in pressure in between 0.3 to 1.2 MPa. As a driving force pressurized liquid ammonia (j) is used. The pressurized carbamate solution enters the reactor (REA) via line (i) together with the feedstock carbon dioxide via line (b). In the reactor (REA) the endothermic urea reactor proceeds approaching the chemical equilibrium. The urea solution comprising urea, ammonia, carbon dioxide and water together with the non-condensed vapor comprising ammonia, carbon dioxide and inerts leave the reactor (REA) via line (a). In this line (a) the urea solution is expanded by a valve to a pressure that is in the range of 0.3 to 1.2 MPa lower than the pressure in the reactor (REA). Said urea solution together with the non-condensed and expanded vapor enters the stripper (STR) at the top side.

The stripped urea solution leaving the stripper (STR) at the bottom via line (d) comprises typically 6 to 10 % by weight of non-converted carbon dioxide and is expanded and subsequently added to a first recovery section (MP REC) operated at a pressure in the range of 1.5 to 2.5 MPa. The non-condensed vapor leaving the synthesis section via line (f) is added to the first recovery section (MP REC) as well. In this first recovery section (MP REC) the excess of ammonia is separated from the urea and carbamate solution. The excess ammonia in the vapor phase leaves the first recovery section via line (k) to the ammonia recovery section (AM REC) in which the ammonia vapor is condensed (1) after which it is collected in an ammonia collection vessel (VESSEL). The feedstock ammonia liquid from battery limit is added to this vessel (VESSEL) as well, via line (h), after which the liquid ammonia via line (j) is increased in pressure and used as driving force for the ejector (EJEC) in the synthesis section.

The carbamate left in the urea solution is heated in a decomposer in the first recovery section (MP REC) to separate the ammonia and carbon dioxide from the urea solution. The formed vapor in said decomposer comprises ammonia and carbon dioxide and is subjected to condensation to form a carbamate solution that leaves the first recovery section (MP REC) via line (n). This carbamate solution is increased in pressure where after it is added to the condenser (HPC) in the synthesis section.

The urea solution leaving the first recovery section (MP REC) via line (m) is expanded where after added to a second recovery section (LP REC) typically operated at a pressure in the range of 0.2 to 0.6 MPa. This urea solution leaving the first recovery section (MP REC) still contains a considerable amount of ammonia and carbon dioxide. The second recovery section (LP REC) comprises usually a decomposer and a condensation. The decomposer is usually a shell and tube heat exchanger. In the decomposer the carbamate left in the urea solution is decomposed into ammonia and carbon dioxide vapor by adding steam to the shell side. The urea solution leaves said decomposer at a temperature of 120 - 145 °C and preferably at a temperature of 130 - 140 °C where after the pressure of said urea solution is decreased causing a further purification of the urea solution by flashing.

The released vapor from the decomposer comprising ammonia, carbon dioxide and water is condensed in the condensation section of this recovery section (LP REC) by the formation of a carbamate solution that is pumped to preferably the condensation in the first recovery section (MP REC) via line (p).

The urea solution leaving the second recovery section (LP REC) arrives via line (o) the evaporation section (EVAP). In this evaporation section (EVAP) the urea solution is concentrated to the desired urea concentration that is determined by the finishing section (not indicated). Usually the concentration of the urea solution takes place at sub-atmospheric pressure and dependent of the required concentration one or a plurality of evaporators are used. The concentrated urea solution leaves the evaporation section via line (r). The released vapor leaving the evaporators via line (q) comprising water, ammonia and carbon dioxide is condensed to form process condensate. Since the operation pressure in the evaporator is sub-atmospheric, small amounts of urea is entrained and leaves the evaporators via the vapor phase (q). This urea is traced back in the formed process condensate leaving the condensation section (COND) via line (s). Said formed process condensate is after totally or partially being used as absorbent in the absorbers of the plant to purify inert vapor, subjected to a process condensate treatment section (TREAT). The process condensate treatment section comprises a first desorber, a hydrolyser column or horizontal vessel followed by a next second desorber. In the first desorber the bulk of ammonia and carbon dioxide is stripped from the water solution. The stripped condensate is subjected to a heated liquid filled column/ vessel (hydrolyser) in which the urea, occurring in said condensate, is decomposed into ammonia and carbon dioxide. The effluent leaving this hydrolyser comprising water, ammonia and carbon dioxide is subjected to the next second desorber in which the left ammonia and carbon dioxide is stripped from the condensate. Usually steam is used as stripping agent in this second desorber. The purified process condensate leaves the treatment section (TREAT) via line (t). The hot off gas leaving the second desorber comprising ammonia and carbon dioxide is usually used as stripping agent for the said first desorber. The off gases leaving the first desorber, comprising ammonia, carbon dioxide and water, are condensed where after that formed concentrated process condensate is pumped via line (u) to the condensation section of the recovery section (LP REC).

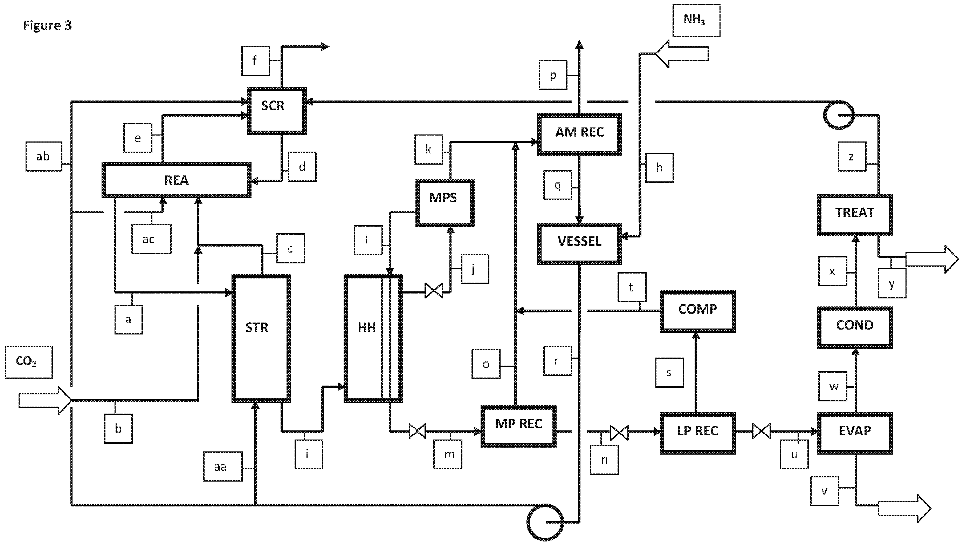

Figure 3 illustrates an embodiment according to the invention. The synthesis section comprises a reactor (REA) that includes a condensation zone, a stripper (STR), a scrubber (SCR) and a high-pressure heat exchanger (HH). The synthesis section is operated at a pressure in the range 18 to 21 MPa and preferably in the range 18.5 to 20.5 MPa and the equipment in the synthesis section are in fluid communication. The synthesis process loop contains gravity flows. The reaction zone (REA) as described in this example refers to a condenser and a reactor combined in one piece of equipment as described in Ullmann's Encyclopedia of Industrial Chemistry, Vol. A27, 1996, pp 333-350. It will be understood that the invention is also applicable for those concepts where the reactor zone is separated from the condensation zone in the synthesis section and consists of separate pieces of equipment.

The urea solution leaving the reaction zone (REA) via line (a), typically controlled at an ammonia to carbon dioxide molar ratio in the range 3.8 to 4.2 mol/mol and preferably in the range 3.9 to 4.1 mol/mol, is added to the stripper (STR) that is counter currently contacted with ammonia. The water to carbon dioxide molar ratio in this urea solution is typically in the range 0.2 to 0.45 mol/mol and preferably in the range 0.25 and 0.4 mol/mol. As a heating agent, steam at a pressure of 1.5 to 2.5 MPa is supplied to the shell side of said stripper (STR) or via steam coils located in said stripper (STR) in order to obtain a urea concentration in the urea solution leaving that stripper of approximately 38 to 50 % by weight.

The stripped vapor leaving the stripper (STR) via line (c) is supplied together with fresh carbon dioxide via line (b) to the condenser part and for a small part to the reaction part of the reactor (REA). In the condenser part of the reactor (REA) the ammonia and carbon dioxide is condensed into a carbamate solution. The released condensation heat is used to form steam that is used for heating purposes and driving force for ejectors in the downstream urea processing sections. Since the reaction zone (REA) is a submerged type, residence time of the liquid phase is created, which causes the endothermic urea reaction to proceed already in the condensation part of said reaction zone. The thus formed solution containing already a substantial amount of formed urea arrives in the reaction part of the reaction zone (REA) where the endothermic urea reaction approaches the equilibrium. The non-condensed vapor comprising ammonia, carbon dioxide, water and inerts leaves said reaction zone (REA) by line (e) and is supplied to the scrubber (SCR). In the scrubber (SCR) the inert containing off gas of the reaction zone (REA) is scrubbed with cold ammonia supplied by line (ab) and/or process condensate supplied via line (z). The inert vapor leaving the scrubber (SCR) via line (f) is released into the atmosphere or further purified in an absorber (not shown). The concentrated condensate leaving the scrubber (SCR) is supplied via line (d) to the condensation part in the reaction zone (REA).

The urea solution leaving the stripper (STR) via line (i) is heat exchanged with the urea solution descending ammonia/urea solution separator (MPS) via line (1). This heat exchange is done directly in one piece of equipment (HH) or can be done in two pieces of equipment by using for instance a tempered condensate system as a heating transfer medium. The cooled urea solution leaving the heat exchanger (HH) via line (j) is expanded to a pressure in the range 1.0 to 3.0 MPa and preferably in the range 1.7 to 2.7 MPa. By this expansion ammonia is released from the urea solution that leaves the ammonia/ urea solution separator (MPS) via line (k) while the urea solution leaves this separator (MPS) via line (l) and is heated in heater (HH).

The urea solution leaving the heater (HH) is discharged to the first recovery section (MP REC) via line (m). The pressure in this first recovery section is slightly below the pressure occurring in the ammonia/ urea solution separator (MPS) in order to obtain a driving force. In the first recovery section the bulk of ammonia is stripped from the urea solution. Usually a stripper column is used and heat is supplied by adding life steam to this column but preferably to use a heater or a coil in order to prevent excessive water entrance to the process. The stripped ammonia vapor comprising ammonia, small amounts of water and traces of carbon dioxide is added via line (o) together with the ammonia rich vapor leaving the ammonia/ urea solution separator (MPS) via line (k) to an ammonia condensation (AM REC). Since the pressure in this section is preferably in the range 1.7 to 2.7 MPa, cooling water can be used to condense the ammonia vapor. The condensed ammonia via line (q) together with the ammonia feedstock via line (h) are collected in an ammonia collecting vessel (VESSEL). The ammonia leaving this ammonia collecting vessel (VESSEL) via line (r) is increased in pressure by usually applying a pump and supplied partly or total to the stripper (STR) via line (aa), partly or total to the reactor (REA) via line (ac) and partly or total to the scrubber (SCR) via line (ab) in the synthesis section. Preferably the ammonia sent to the stripper (STR) via line (aa) is evaporated prior to the injection into the stripper bottom.

The urea solution leaving the first recirculation section, comprising still a substantial amount of ammonia, is sent via line (n) to a next second recirculation section (LP REC) that is operated at a pressure in the range 0.1 to 0.6 MPa and preferably in the range 0.2 to 0.5 MPa. Also in this second recirculation section (LP REC) the ammonia is stripped from the urea solution. Usually a stripper column is used and heat is supplied by adding life steam to this column but preferably to use a heater or a coil in order to prevent excessive water entrance to the process. The stripped ammonia vapor comprising ammonia, small amounts of water and traces of carbon dioxide is added via line (s) to an ammonia compressor (COMP) that increases the ammonia vapor pressure and subsequently conveys the ammonia vapor to the ammonia condenser (AM REC) via line (t).

The urea solution leaving the second recovery section (LP REC) arrives via line (u) the evaporation section (EVAP). In this evaporation section (EVAP) the urea solution is concentrated to the desired urea concentration that is determined by the finishing section (not indicated). Usually the concentration of the urea solution takes place at sub-atmospheric pressure and dependent of the required concentration one or a plurality of evaporators are used. The concentrated urea solution leaves the evaporation section via line (v). The released vapor leaving the evaporators via line (w) comprising water, ammonia and carbon dioxide is condensed to form process condensate. Since the operation pressure in the evaporator is sub-atmospheric, small amounts of urea is entrained and leaves the evaporators via the vapor phase. This urea is traced back in the formed process condensate leaving the condensation section (COND) via line (x). Said formed process condensate is after totally or partially being used as absorbent in the absorbers of the plant to purify inert vapor, subjected to a process condensate treatment section (TREAT). The process condensate treatment section comprises a first desorber, a hydrolyser column or horizontal vessel followed by a next second desorber. In the first desorber the bulk of ammonia and residual carbon dioxide is stripped from the water solution. The stripped condensate is subjected to a heated liquid filled column/ vessel (hydrolyser) in which the urea, occurring in said condensate, is decomposed into ammonia and carbon dioxide. The effluent leaving this hydrolyser comprising water, ammonia and carbon dioxide is subjected to the next second desorber in which the left ammonia and carbon dioxide is stripped from the condensate. Usually steam is used as stripping agent in this second desorber. The purified process condensate leaves the treatment section (TREAT) via line (y). The hot off gas leaving the second desorber comprising ammonia and carbon dioxide is usually used as stripping agent for the said first desorber. The off gases leaving the first desorber, comprising ammonia, carbon dioxide and water, are condensed where after that formed process condensate is pumped via line (z) to preferably the scrubber (SCR) in the synthesis section.

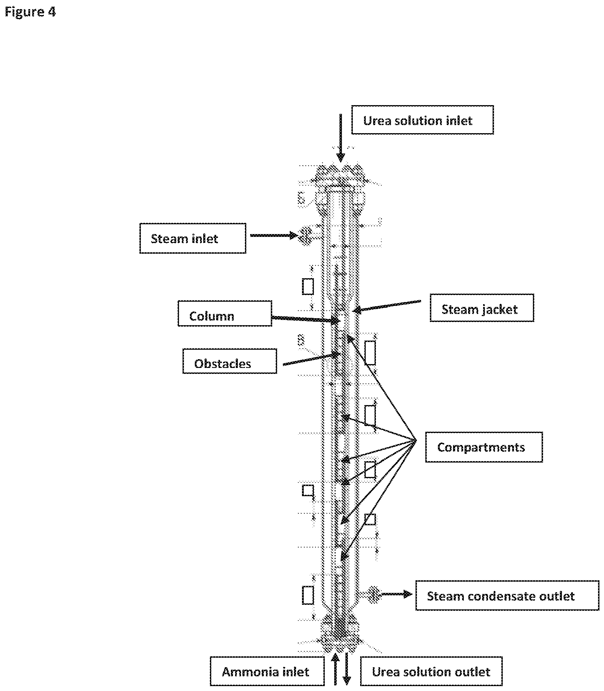

Figure 4 schematically shows an aerosol stripper that can be used in the present invention. In the aerosol stripper the urea solution, added at the top, is distributed across one or more columns. The figure shows one column for illustrating the principle. The column comprises a number of compartments where the vapor is added at the bottom and the urea solution is overflowing by one or more down comers from the one compartment to the other. In each compartment a gas/liquid mixture is obtained to form an aerosol in which the liquid phase is the dispersed phase while the vapor forms the continuous phase. To prevent aerosol carry over from the one to the other compartment, obstacles to break the aerosol are located at the top of each compartment in the column. The number of compartments in the column is in the range 2-20 but preferably in the range 3-10. In the preferred embodiment of the aerosol stripper, ammonia is added with the aim to suppress the biuret equilibrium reaction according the following equation:

$$2NH_2 - CO - NH_2 \leftrightarrow NH_2 - CO - NH - CO -NH_2 +NH_3$$

As shown in this equation biuret formation is reduced in an ammonia environment. As a result the biuret content in the urea solution leaving the preferred embodiment of the aerosol stripper is reduced by at least 0.1 % by weight as compared to the biuret content in usual strippers where no ammonia is added to the stripper bottom.

The heat required to evaporate the non-converted ammonia and carbon dioxide from the urea solution is supplied via a jacket around the column or coils in the column. In the preferred embodiment the aerosol stripper comprises as number of columns dependent of the required plant capacity and these columns are positioned in a shell where steam for heating is supplied.

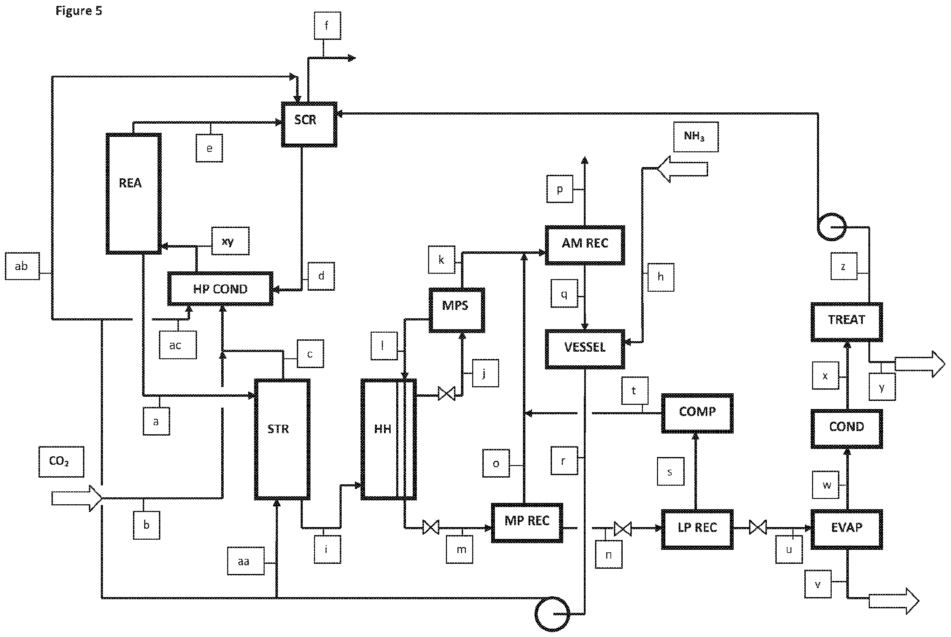

Figure 5 shows another embodiment according to the invention. In this embodiment the reactor (REA) and the condenser (HP COND) are separate pieces of equipment. The flow (xy) from the condenser contains both gas and liquid and is supplied to the reactor. The remaining parts of the scheme are the same as in Figure 4.

**Claims**

1. A process for urea production in a urea plant comprising a synthesis section, said section comprising a urea reactor, a stripper, a condenser and optionally a scrubber, and a recovery section operating at lower pressure than the synthesis section, said process comprising the steps of:

a) reacting ammonia and $CO_2$ under urea forming conditions in the urea reactor to obtain a first urea solution,
b) stripping the first urea solution with ammonia in the stripper to form a second urea solution and a first vapor phase containing ammonia, $CO_2$ and water,
c) condensing the first vapor phase obtained in b) in the condenser to form a carbamate solution which is recycled to the reactor,
d) subjecting the second urea solution to ammonia recovery in the recovery section to produce a third urea solution and a second vapor phase containing ammonia,
e) recycling the second vapor phase to the urea synthesis section, wherein in the synthesis section a substantially isobaric loop is formed,

wherein the second urea solution contains less than 3 wt.%, preferably less than 2 wt.%, more preferably less than 1 wt.% of $CO_2$, and
wherein the condenser is a submerged condenser.

2. The process according to claim 1, wherein all the flows in the synthesis section are gravity flows.

3. The process according to claim 1 or 2, wherein the second urea solution is subjected to flashing under medium pressure to obtain a flashed urea solution and an ammonia containing vapor.

4. The process to any of the preceding claims, wherein the stripper is an aerosol stripper.

5. The process according to claim 3, wherein the second urea solution is cooled by indirect heat exchange with said flashed urea solution.

6. The process according to any of the preceding claims wherein the amount of water in the second vapor phase recycled to the reactor is less than 10% by weight.

7. The process according to any of the preceding claims wherein the water to $CO_2$ ratio in the second urea solution is in the range 0.2-0.45 mol/mol and preferably in the range 0.25-0.4 mol/mol.

8. The process according to any of the preceding claims wherein the urea reactor and the urea condenser are combined in one apparatus.

9. The process according to any of the preceding claims wherein the N/C ratio in step (a) is in the range 3.8-4.2, preferably in the range 3.9-4.1.

10. The process according to any of the preceding claims wherein the pressure in the reactor is in the range 18-21 MPa, preferably 18.5-20.5 MPa.

11. The process according to any one of the preceding claims, wherein the submerged condenser is a horizontal submerged condenser.

**Patentansprüche**

1. Verfahren zur Harnstoff Herstellung in einer Harnstoffanlage, umfassend einen Syntheseabschnitt, der Abschnitt umfassend einen Harnstoffreaktor, einen Stripper, einen Kondensator und optional einen Gaswäscher, und einen Rückgewinnungsabschnitt, arbeitend bei niedrigerem Druck als der Syntheseabschnitt, das Verfahren umfassend die Schritte von:

a) Reagieren von Ammoniak und $CO_2$ unter Harnstoff bildenden Bedingungen in dem Harnstoffreaktor, um eine erste Harnstofflösung zu erhalten,

b) Strippen der ersten Harnstofflösung mit Ammoniak in dem Stripper, um eine zweite Harnstofflösung und eine erste Dampfphase, enthaltend Ammoniak, $CO_2$ und Wasser, auszubilden,

c) Kondensieren der ersten Dampfphase, erhalten in b), in dem Kondensator, um eine Carbamatlösung auszubilden, die in den Reaktor rückgeführt wird,

d) Aussetzen der zweiten Harnstofflösung einer Ammoniakrückgewinnung in dem Rückgewinnungsabschnitt, um eine dritte Harnstofflösung und eine zweite Dampfphase, enthaltend Ammoniak, herzustellen,

e) Rückführen der zweiten Dampfphase zu dem Harnstoffsyntheseabschnitt, wobei in dem Syntheseabschnitt eine im Wesentlichen isobare Schleife ausgebildet wird,

wobei die zweite Harnstofflösung weniger als 3 Gew.-%, vorzugsweise weniger als 2 Gew.-%, bevorzugter weniger als 1 Gew.-% $CO_2$ enthält, und

wobei der Kondensator ein Tauchkondensator ist.

2. Verfahren nach Anspruch 1, wobei all die Strömungen in dem Syntheseabschnitt Schwerkraftströmungen sind.

3. Verfahren nach Anspruch 1 oder 2, wobei die zweite Harnstofflösung einem Überfangen unter mittlerem Druck ausgesetzt wird, um eine überfangene Harnstofflösung und einen Ammoniak enthaltenden Dampf zu erhalten.

4. Verfahren nach einem der vorhergehenden Ansprüche, wobei der Stripper ein Aerosol-Stripper ist.

5. Verfahren nach Anspruch 3, wobei die zweite Harnstofflösung durch indirekten Wärmeaustausch mit der überfangenen Harnstofflösung gekühlt wird.

6. Verfahren nach einem der vorhergehenden Ansprüche, wobei die Menge an Wasser in der zweiten Dampfphase, rückgeführt in den Reaktor, weniger als 10 Gewichtsprozent ist.

7. Verfahren nach einem der vorhergehenden Ansprüche, wobei das Wasser zu $CO_2$ Verhältnis in der zweiten Harnstofflösung in dem Bereich 0,2 - 0,45 mol/mol und vorzugsweise in dem Bereich 0,25 - 0,4 mol/mol ist.

8. Verfahren nach einem der vorhergehenden Ansprüche, wobei der Harnstoffreaktor und der Harnstoffkondensator in einer Vorrichtung kombiniert sind.

9. Verfahren nach einem der vorhergehenden Ansprüche, wobei das N/C-Verhältnis in Schritt (a) in dem Bereich 3,8 - 4,2, vorzugsweise in dem Bereich 3,9 - 4,1 ist.

10. Verfahren nach einem der vorhergehenden Ansprüche, wobei der Druck in dem Reaktor in dem Bereich 18 - 21 MPa, vorzugsweise 18,5 - 20,5 MPa ist.

11. Verfahren nach einem der vorhergehenden Ansprüche, wobei der Tauchkondensator ein horizontaler Tauchkondensator ist.

**Revendications**

1. Procédé de production d'urée dans une usine d'urée comprenant une section de synthèse, ladite section comprenant un réacteur d'urée, un épurateur, un condenseur et éventuellement un laveur, et une section de récupération fonctionnant à une pression plus faible que la section de synthèse, ledit procédé comprenant les étapes de :

a) réaction d'ammoniac et de $CO_2$ dans des conditions de formation d'urée dans le réacteur d'urée pour obtenir une première solution d'urée,

b) épuration de la première solution d'urée avec de l'ammoniac dans l'épurateur pour former une seconde solution d'urée et une première phase vapeur contenant de l'ammoniac, $CO_2$ et de l'eau,

c) condensation de la première phase vapeur obtenue dans b) dans le condenseur pour former une solution de carbamate qui est recyclée dans le réacteur,

d) soumission de la seconde solution d'urée à une récupération d'ammoniac dans la section de récupération pour produire une troisième solution d'urée et une seconde phase vapeur contenant de l'ammoniac,

e) recyclage de la seconde phase vapeur dans la section de synthèse d'urée,

dans lequel dans la section de synthèse une boucle pratiquement isobare est formée,
dans lequel la seconde solution d'urée contient moins de 3 % en masse, de préférence moins de 2 % en masse, encore mieux moins de 1 % en masse de $CO_2$, et
dans lequel le condenseur est un condenseur submergé.

2. Procédé selon la revendication 1, dans lequel tous les écoulements dans la section de synthèse sont des écoulements par gravité.

3. Procédé selon la revendication 1 ou 2, dans lequel la seconde solution d'urée est soumise à un traitement flash sous pression moyenne pour obtenir une solution d'urée flashée et une vapeur contenant de l'ammoniac.

4. Procédé selon l'une quelconque des revendications précédentes, dans lequel l'épurateur est un épurateur à aérosol.

5. Procédé selon la revendication 3, dans lequel la seconde solution d'urée est refroidie par échange de chaleur indirecte avec ladite solution d'urée flashée.

6. Procédé selon l'une quelconque des revendications précédentes, dans lequel la quantité d'eau dans la seconde phase vapeur recyclée dans le réacteur est inférieure à 10 % en masse.

7. Procédé selon l'une quelconque des revendications précédentes, dans lequel le rapport eau à $CO_2$ dans la seconde solution d'urée se trouve dans l'intervalle de 0,2 - 0,45 mol/mol et de préférence dans l'intervalle de 0,25 - 0,4 mol/mol.

8. Procédé selon l'une quelconque des revendications précédentes, dans lequel le réacteur d'urée et le condenseur d'urée sont combinés en un appareil.

9. Procédé selon l'une quelconque des revendications précédentes, dans lequel le rapport N/C dans l'étape (a) se trouve dans l'intervalle de 3,8 - 4,2, de préférence dans l'intervalle de 3,9 - 4,1.

10. Procédé selon l'une quelconque des revendications précédentes, dans lequel la pression dans le réacteur se trouve dans l'intervalle de 18 - 21 MPa, de préférence 18,5 - 20,5 MPa.

11. Procédé selon l'une quelconque des revendications précédentes, dans lequel le condenseur submergé est un condenseur submergé horizontal.

Figure 1

Figure 2

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*


**Patent documents cited in the description**

- GB 1188051 A
- US 20110160486 A1

- US 6730811 B1
- CA 975797


**Non-patent literature cited in the description**

- Ullmann's Encyclopedia of Industrial Chemistry. 1996, vol. A27, 333-350

- Ullmann's Encyclopedia of Industrial Chemistry. 1996, vol. A27, 336-338